(19) Europäisches Patentamt
European Patent Office
Office européen des brevets

(11) Veröffentlichungsnummer: **0 438 575 B1**

(12) **EUROPÄISCHE PATENTSCHRIFT**

(45) Veröffentlichungstag der Patentschrift: **26.10.94**

(51) Int. Cl.5: **C07C 43/174**, C09K 19/30, C09K 19/12, C07C 43/225, C07C 69/74, C07C 255/50

(21) Anmeldenummer: **90912438.0**

(22) Anmeldetag: **08.08.90**

(86) Internationale Anmeldenummer:
**PCT/EP90/01292**

(87) Internationale Veröffentlichungsnummer:
**WO 91/02710 (07.03.91 91/06)**

Die Akte enthält technische Angaben, die nach dem Eingang der Anmeldung eingereicht wurden und die nicht in dieser Patentschrift enthalten sind.

# (54) BENZOLDERIVATE UND FLÜSSIGKRISTALLINES MEDIUM.

(30) Priorität: **12.08.89 DE 3926746**
**12.08.89 DE 3926749**
**31.08.89 DE 3928818**
**09.01.90 DE 4000392**
**22.01.90 DE 4001685**
**13.03.90 DE 4007864**
**28.03.90 DE 4009908**

(43) Veröffentlichungstag der Anmeldung:
**31.07.91 Patentblatt 91/31**

(45) Bekanntmachung des Hinweises auf die Patenterteilung:
**26.10.94 Patentblatt 94/43**

(84) Benannte Vertragsstaaten:
**DE FR GB IT NL**

(73) Patentinhaber: **MERCK PATENT GmbH**
**Postfach,**
**Frankfurter Strasse 250**
**D-64271 Darmstadt (DE)**

(72) Erfinder: **WÄCHTLER, Andreas**
**Goethestrasse 34**
**D-6103 Griesheim (DE)**
Erfinder: **HITTICH, Reinhard**
**Am Kirchberg 11**
**D-6101 Modautal 1 (DE)**
Erfinder: **POETSCH, Eike**
**Am Buchwald 4**
**D-6109 Mühltal 6 (DE)**
Erfinder: **PLACH, Herbert**
**Wingertsbergstr. 5**
**D-6100 Darmstadt (DE)**
Erfinder: **COATES, David**
**87 Sopwith Crescent**
**Merley**
**Wimborne Dorset BH21 3SW (GB)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 438 575 B1

(56) Entgegenhaltungen:
**EP-A- 0 168 683** **EP-A- 0 258 868**
**EP-A- 0 272 580** **EP-A- 0 410 469**
**EP-A- 0 415 090** **DE-A- 3 237 020**
**DE-A- 3 631 611**

**PATENT ABSTRACTS OF JAPAN, vol. 8, no. 145 (C-232)(1582), 6 July 1984; & JP-A-59 53 459**

Erfinder: **FINKENZELLER, Ulrich**
**Waldpfad 74**
**D-6831 Plankstadt (DE)**
Erfinder: **GEELHAAR, Thomas**
**Trajanstrasse 12**
**D-6500 Mainz (DE)**
Erfinder: **REIFFENRATH, Volker**
**Jahnstrasse 18**
**W-6101 Rossdorf (DE)**
Erfinder: **RIEGER, Bernhard**
**Hauptstrasse 31a**
**D-6115 Münster-Altheim (DE)**

## Beschreibung

Die Erfindung betrifft neue Benzolderivate der Formel I

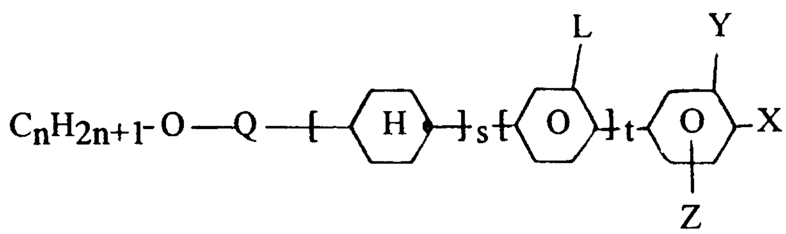

I

worin n 1 bis 7, Q-$(CH_2)_r$-, wobei r 2, 3, 4 oder 5 ist, s 0, 1 oder 2, t 0 oder 1, wobei s + t ≧ 1, X F, Cl, $-CF_3$, -CN, $-OCF_3$ oder $-OCHF_2$ und Y, L und Z jeweils unabhängig voneinander H oder F bedeuten, wobei im Falle X = -CN s 1 oder 2 und/oder Y = F bedeutet, wobei im Hinblick auf die Offenbarungen der Zwischendokumente EP-A-0 410 469 und EP-A-0 415 090 für die Vertragsstaaten DE und GB die folgenden Verbindungen ausgeschlossen sind:

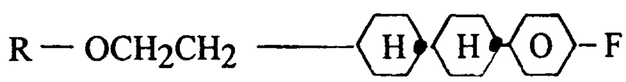

R = geradkettiges Alkyl mit 1 bis 7 C-Atomen

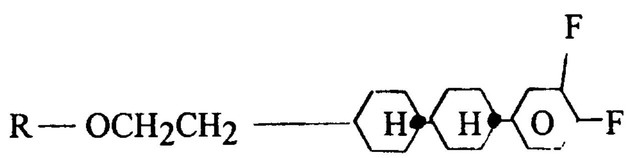

R = geradkettiges Alkyl mit 1 bis 7 C-Atomen

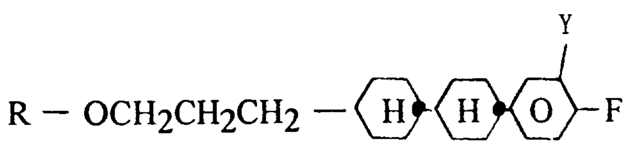

R = geradkettiges Alkyl mit 1 bis 7 C-Atomen
Y = Wasserstoff oder Fluor

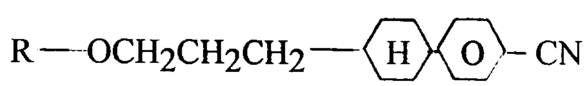

R = geradkettiges Alkyl mit 1 bis 5 C-Atomen

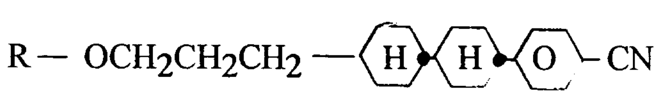

R = geradkettiges Alkyl mit 1 bis 5 C-Atomen

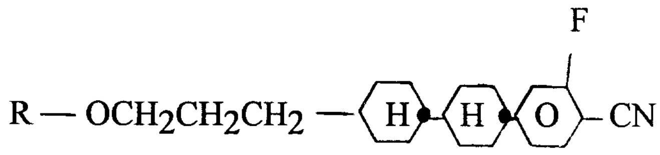

R = geradkettiges Alkyl mit 1 bis 5 C-Atomen

sowie ein flüssigkristallines Medium für elektrooptische Anzeigen mit mindestens zwei flüssigkristallinen Komponenten, dadurch gekennzeichnet, daß mindestens eine Komponente ein Benzolderivat der Formel I ist,

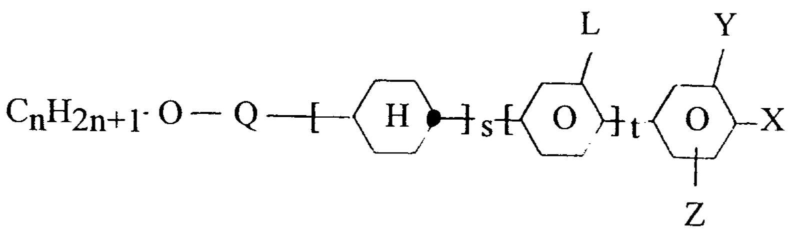 I

worin n 1 bis 7, Q $-(CH_2)_r-$, wobei r = 1, 2, 3, 4 oder 5 ist, s 0, 1 oder 2, t 0 oder 1, wobei s + t ≧ 1, X F, Cl, $-CF_3$, -CN, $-OCF_3$ oder $-OCHF_2$ und Y, L und Z jeweils unabhängig voneinander H oder F bedeuten, und das Medium als weiteren Bestandteil eine oder mehrere Verbindungen der Formeln 1, 2, 3, 4 oder 5 enthält:

R'-L-E-R" 1

R'-L-COO-E-R" 2

R'-L-OOC-E-R" 3

R'-L-$CH_2CH_2$-E-R" 4

R'-L-C ≡ C-E-R" 5

worin L und E, die gleich oder verschieden sein können, jeweils unabhängig voneinander einen bivalenten Rest aus der aus -Phe-, -Cyc-, -Phe-Phe-, - Phe-Cyc-, -Cyc-Cyc-, -Pyr-, -Dio-, -G-Phe-, und -G-Cyc- sowie deren Spiegelbilder gebildeten Gruppen, wobei Phe unsubstituiertes oder durch Fluor substituiertes 1,4-Phenylen,Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten, R' Alkyl, Alkenyl, Alkoxy, Alkenyloxy oder Alkanoyloxy mit jeweils bis zu 8 Kohlenstoffatomen und R" F, Cl, $-CF_3$, $-OCHF_2$ oder $-OCF_3$ bedeutet.

Aus der EP-OS- 0 058 981 sind Flüssigkristalle bekannt, die einen Rest der Formel $-CH_2-O-C_mH_{2m+1}$ - (m = 1 bis 12) tragen. Die dort beschriebenen Verbindungen tragen jedoch keine fluorhaltigen Reste und stellen ganz überwiegend Flüssigkristalle mit mehr oder weniger neutraler dieletrischer Anisotropie dar. Als dielektrisch positiver Flüssigkristall ist lediglich eine Verbindung der Formel

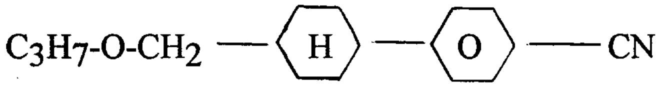

beschrieben. Aus der JP 57-108 056-A und der JP-A- 5953459 sind Flüssigkristalle der Formel

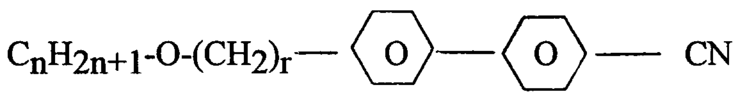

bekannt (n = 1 bis 9 bzw. 1 bis 6; r = 1, 2 oder 3).

Aus der DE-A-32 37 020 sind Flüssigkristalle der Formel

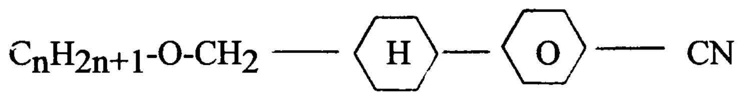

bekannt (n = 1 bis 8).

Derartige Verbindungen werden jedoch nicht den hohen Anforderungen an den elektrischen Widerstand gerecht, wie sie beispielsweise für Anzeigen mit aktiver Matrix gefordert werden. Darüberhinaus zeigen solche Verbindungen eine recht hohe Temperaturabhängigkeit der Schwellenspannung und für Benzonitrile ungewöhnlich niedrige Klärpunkte.

Eine Verbindung der Formel

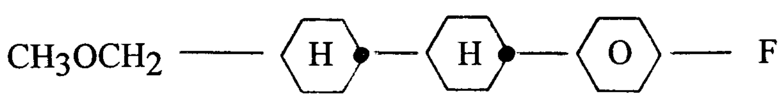

schließlich ist aus DE-A-36 31 611 bekannt.

Die Verbindungen der Formel I können wie ähnliche, z.B. aus der DE-OS 26 36 684 bzw. 23 56 085 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand insbesondere unter Temperatur- und/oder UV-Belastung, ungünstige elastische Eigenschaften für die jeweilige Anwendung, zu hohe Temperaturabhängigkeit der Schwellenspannung.

Insbesondere bei Anzeigen von Supertwisttyp (STN) mit Verdrillungswinkeln von deutlich mehr als 220° oder bei Anzeigen mit aktiver Matrix weisen die bisher eingesetzten Materialien Nachteile auf.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formel I bzw. von flüssigkristallienen Medien enthaltend Verbindungen der Formel I gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit weiten nematischen Bereichen, hervorragender Nematogenität bis zu tiefen Temperaturen, hervorragender chemischer Stabilität, herausragenden elastischen Eigenschaften, ausgeprägtem $\epsilon_{\|}$ bei positiver dieelektrischer Anisotropie, geringer Temperaturabhängigkeit der Schwellenspannung und/oder kleiner optischer Anisotropie erhältlich. Die neuen Verbindungen zeigen außerdem eine gute Löslichkeit für andere Komponenten derartiger Medien und hohe positive dielektrische Anisotropie bei gleichzeitig günstiger Viskosität.

Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität. Durch geeignete Wahl von r und n lassen sich bei beiden Anzeigetypen die Schwellenspannungen deutlich erniedrigen.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie die Verwendung der Verbindungen der Formel I als Komponenten flüssigkristalliner Medien, flüssigkristalline Medien mit einem

Gehalt an mindestens einer Verbindung der Formel I und elektrooptische Anzeigen, die derartige Medien enthalten.

Vor- und nachstehend haben r, Q, n, s, t, L, X, Y und Z die angegebene Bedeutung, sofern nicht ausdrücklich etwas anderes vermerkt ist.

Im Falle X = CN ist vorzugsweise s = 2 und t = 0 oder s = 1 und t = 1 (wobei L H oder vorzugsweise F bedeutet).

In den Verbindungen der Formel I sind die Alkylgruppen $C_nH_{2n+1}$ vorzugsweise geradkettig. Dementsprechend bedeutet $C_nH_{2n+1}$ vorzugsweise Methyl, Ethyl, n-Propyl, n-Butyl, n-Pentyl, n-Hexyl oder n-Heptyl. n ist vorzugsweise 1, 2, 3, 4 oder 5, insbesondere bevorzugt 1. r ist 2, 3, 4 oder 5, bevorzugt 2 oder 3. In flüssigkristallinen Medien gemäß Anspruch 8 kann r auch 1 bedeuten, insbesondere wenn s + t ≧ 2.

Verbindungen der Formel I mit verzweigten Alkylgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methylpropyl), Isobutyl (= 2-Methyl-propyl), 2-Methyl-butyl, Isopentyl (= 3-Methylbutyl), 2-Methylpentyl, 3-Methylpentyl oder 2-Heptyl (= 1-Methylhexyl).

Der Rest

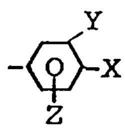

ist vorzugsweise

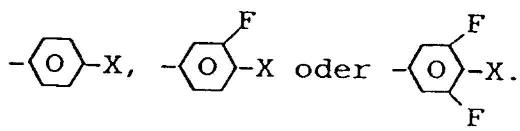

X ist vorzugsweise F, Cl, $-CF_3$,$-OCHF_2$ oder $-OCF_3$.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Zur Synthese der erfindungsgemäßen Verbindungen geeignete Vorstufen sind beispielsweise nach folgendem Syntheseschema erhältlich:

Schema 1a:

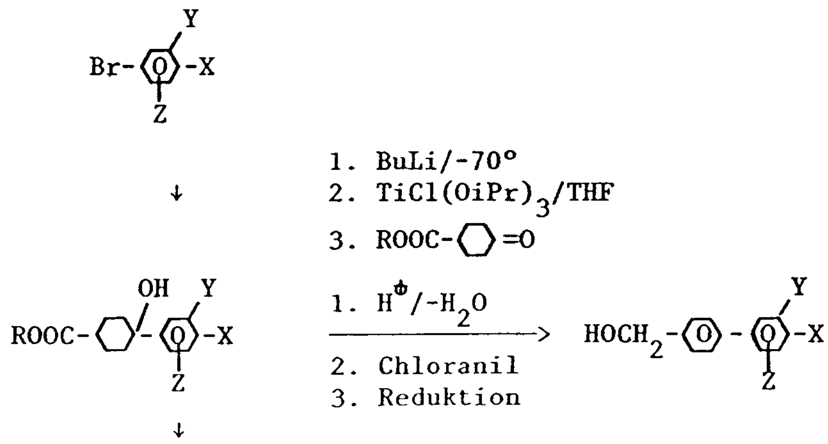
1. BuLi/-70°
2. TiCl(OiPr)3/THF
3. ROOC-
1. H⊕/-H2O
2. Chloranil
3. Reduktion

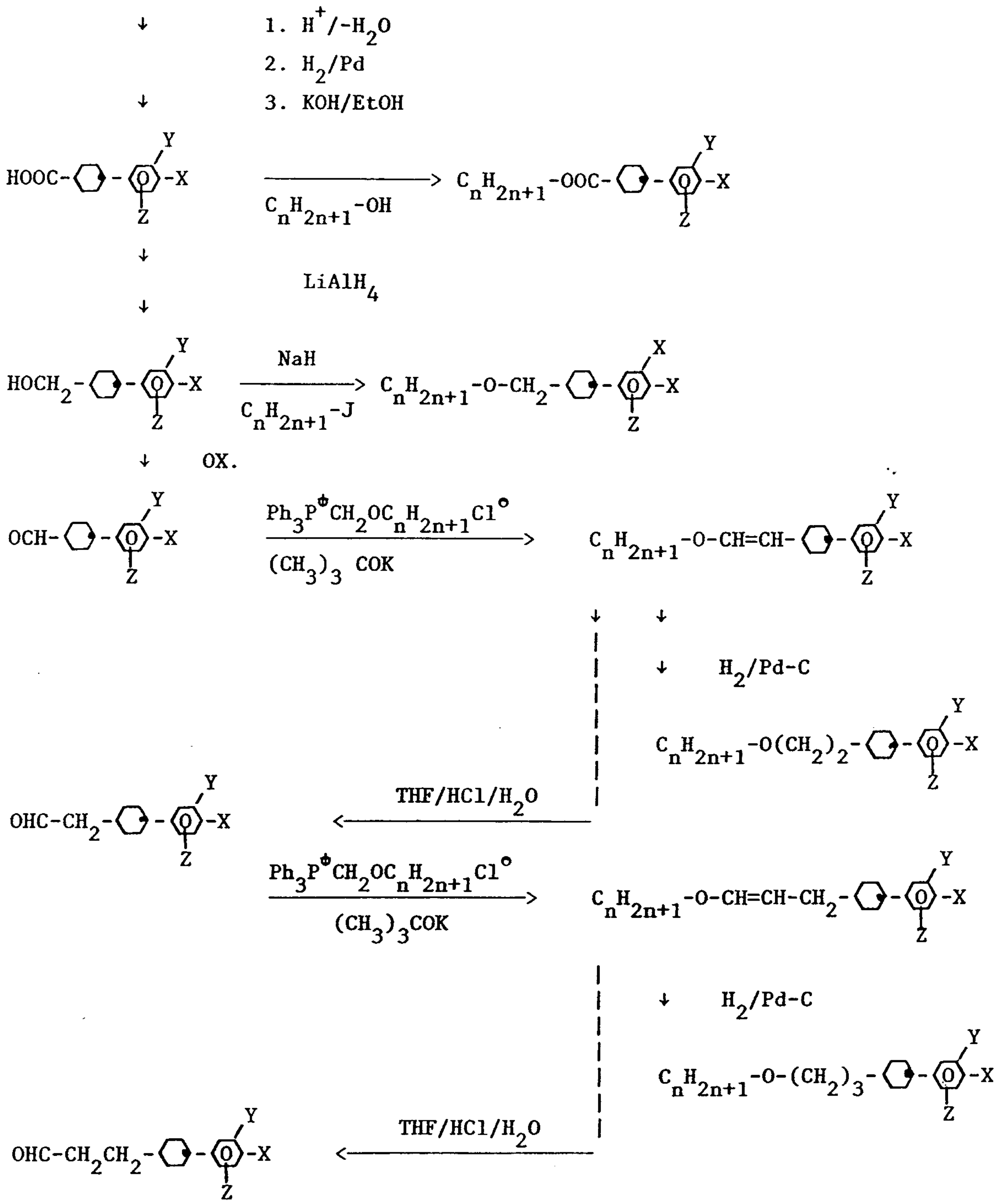

1. $H^+/-H_2O$
2. $H_2/Pd$
3. KOH/EtOH
$C_nH_{2n+1}-OH$
$LiAlH_4$
NaH
$C_nH_{2n+1}-J$
OX.
$Ph_3P^{\oplus}CH_2OC_nH_{2n+1}Cl^{\ominus}$
$(CH_3)_3$ COK
$H_2/Pd-C$
$THF/HCl/H_2O$
$Ph_3P^{\oplus}CH_2OC_nH_{2n+1}Cl^{\ominus}$
$(CH_3)_3COK$
$H_2/Pd-C$
$THF/HCl/H_2O$

Schema 1b:

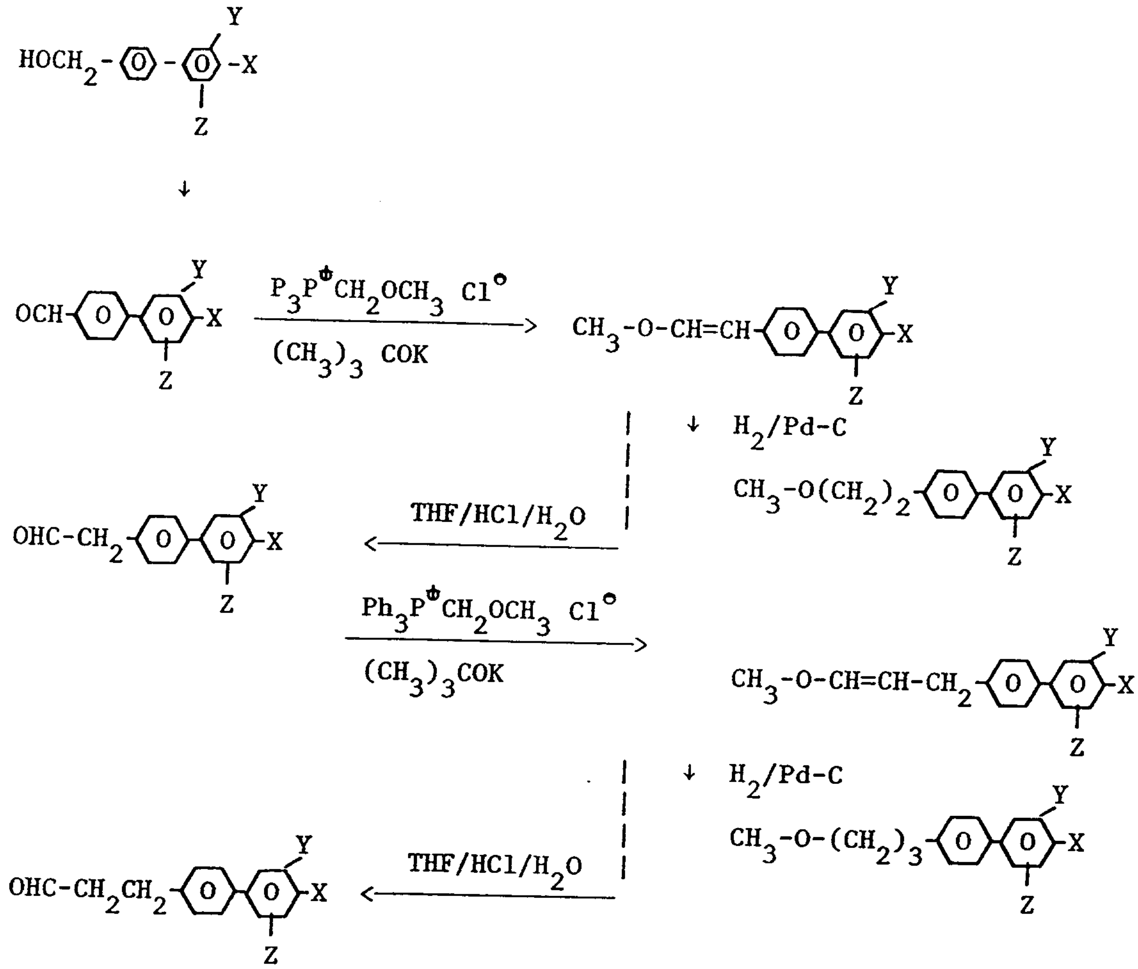


Durch sinngemäße Wiederholung dieser Reaktionssequenz sind die Verbindungen mit r = 4 bzw. 5 erhältlich.

Die aus dem entsprechenden Brombenzol-Derivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Hydrierung der Doppelbindung und Isomerisierung erhält man nach üblichen Methoden den trans-Cyclohexancarbonsäureester. Aus letzterem erhält man nach üblichen Standardverfahren die geeigneten Aldehyde für die erfindungsgemäßen Verbindungen, die aus diesen durch Wittig-Synthese und nachfolgender Hydrierung der Doppelbindung erhältlich sind.

Die als Ausgangsstoffe verwendete Brombenzolderivate sind zum Teil bekannt, zum Teil können sie ohne Schwierigkeiten nach Standardverfahren der organischen Chemie aus literaturbekannten Verbindungen hergestellt werden. Beispielsweise sind die $OCF_3$- oder $OCHF_2$-Verbindungen nach bekannten Verfahren aus den entsprechenden Phenolen bzw. die $CF_3$- oder CN-Verbindungen aus den entsprechenden Benzoesäuren erhältlich. Verbindungen der Formel

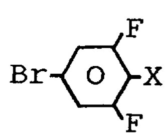

oder auch entsprechende monofluorierte Verbindungen sind beispielsweise aus den bekannten Vorstufen mit X = H durch Lithiierung bei tiefen Temperaturen und anschließende Umsetzung mit einem geeigneten Elektrophil erhältlich.

Die in obigem Reaktionsschema angegebene Homologisierung kann auch nach anderen, dem Fachmann bekannten, Standard-Verfahren erfolgen.

Die Synthese der erfindungsgemäßen Verbindungen mit s = 2 und t = 0 erfolgt nach folgendem Syntheseschema (R = n-Alkyl):

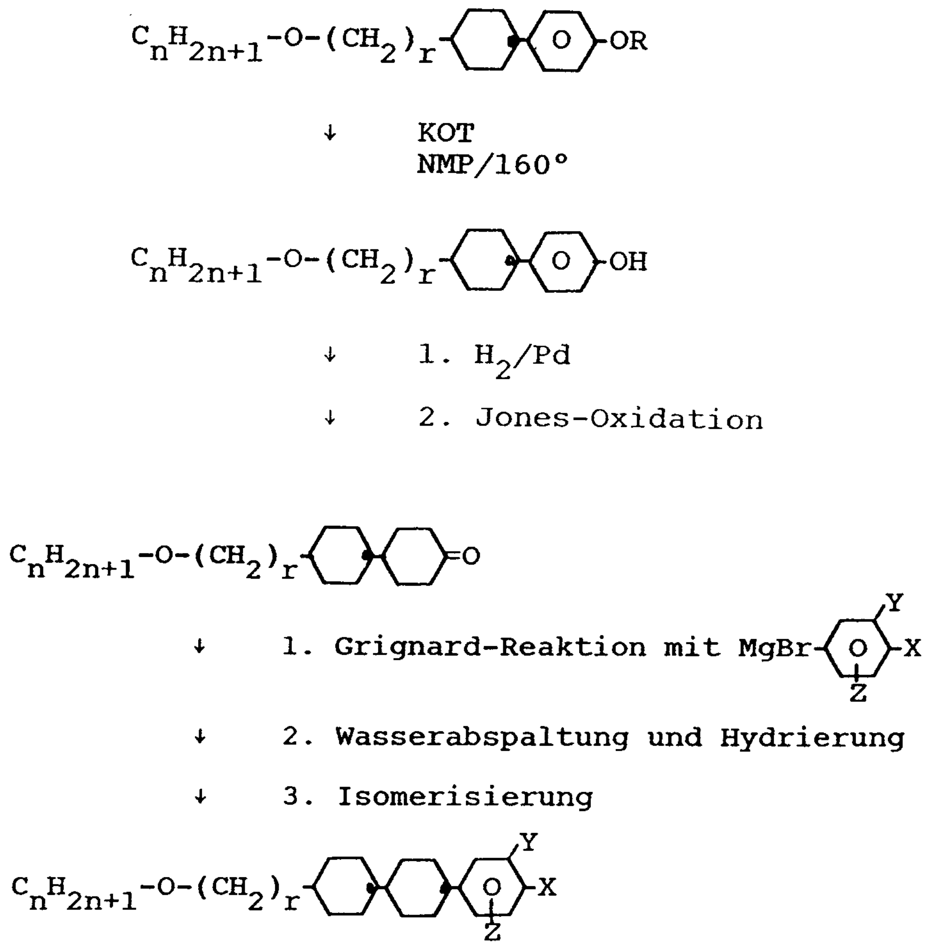

Die Ausgangsmaterialien sind wie folgt erhältlich:

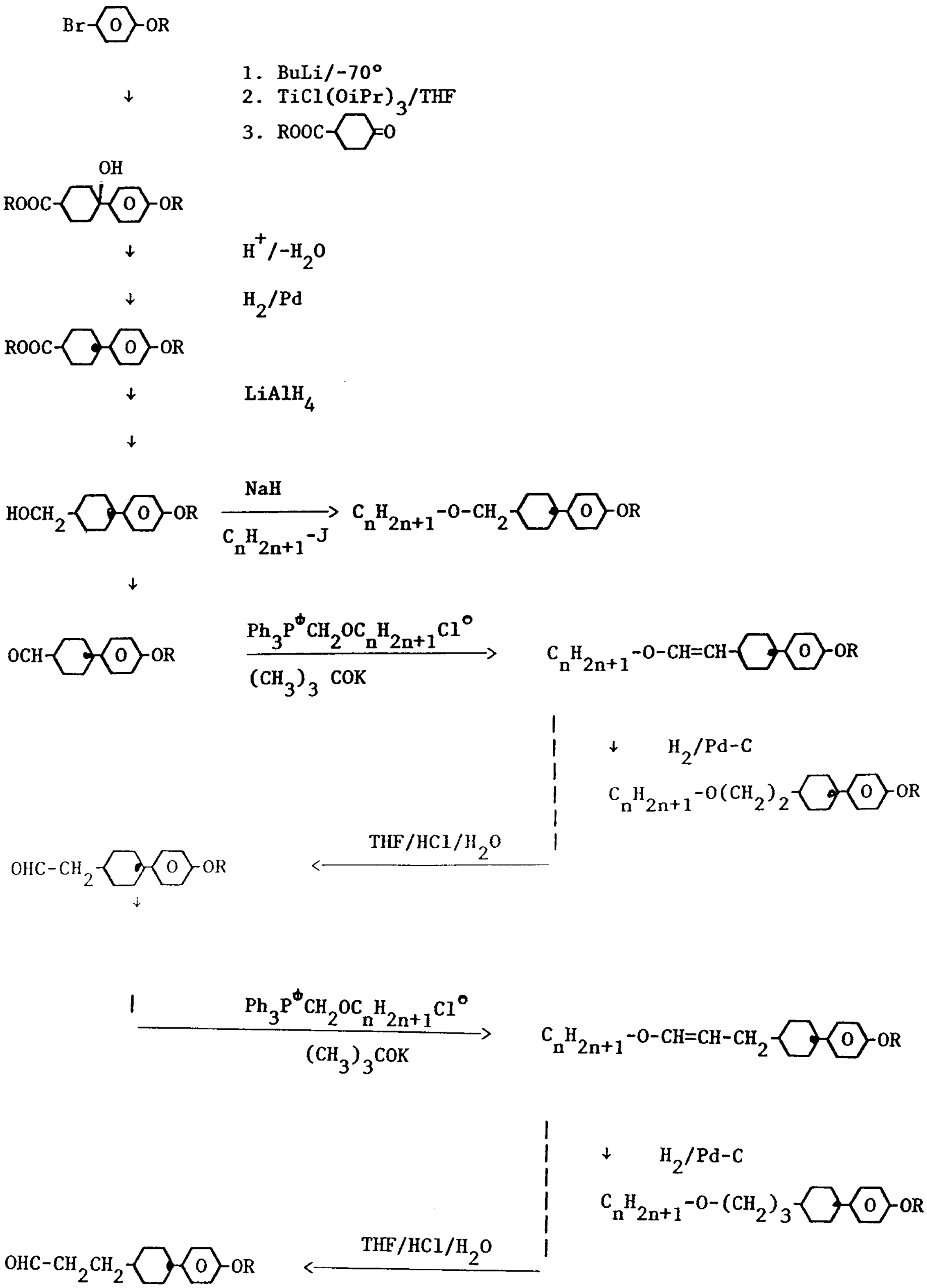


Durch sinngemäße Wiederholung dieser Reaktionssequenz sind die Verbindungen mit r = 4 bzw. 5 erhältlich.

Eine weitere Möglichkeit zur Herstellung der bevorzugten Verbindungen mit r = 3 und n = 1 ist im folgenden aufgezeigt:

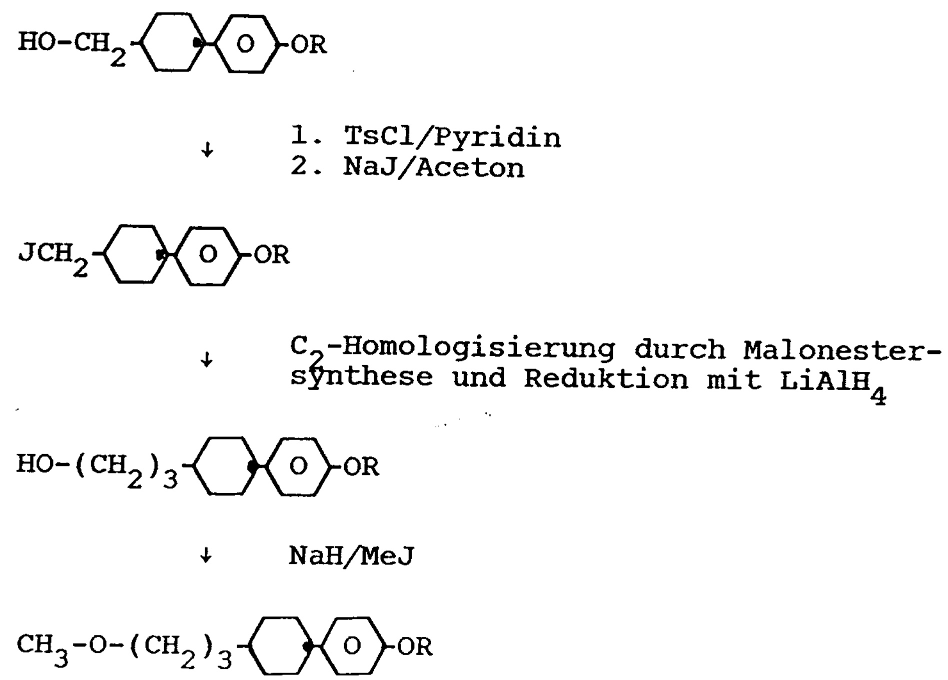


Eine weitere Synthesemöglichkeit für Verbindungen mit s = 2 und t = 0 sowie eine Synthesemöglichkeit für Verbindungen mit s = t = 1 ist folgendem Schema zu entnehmen:

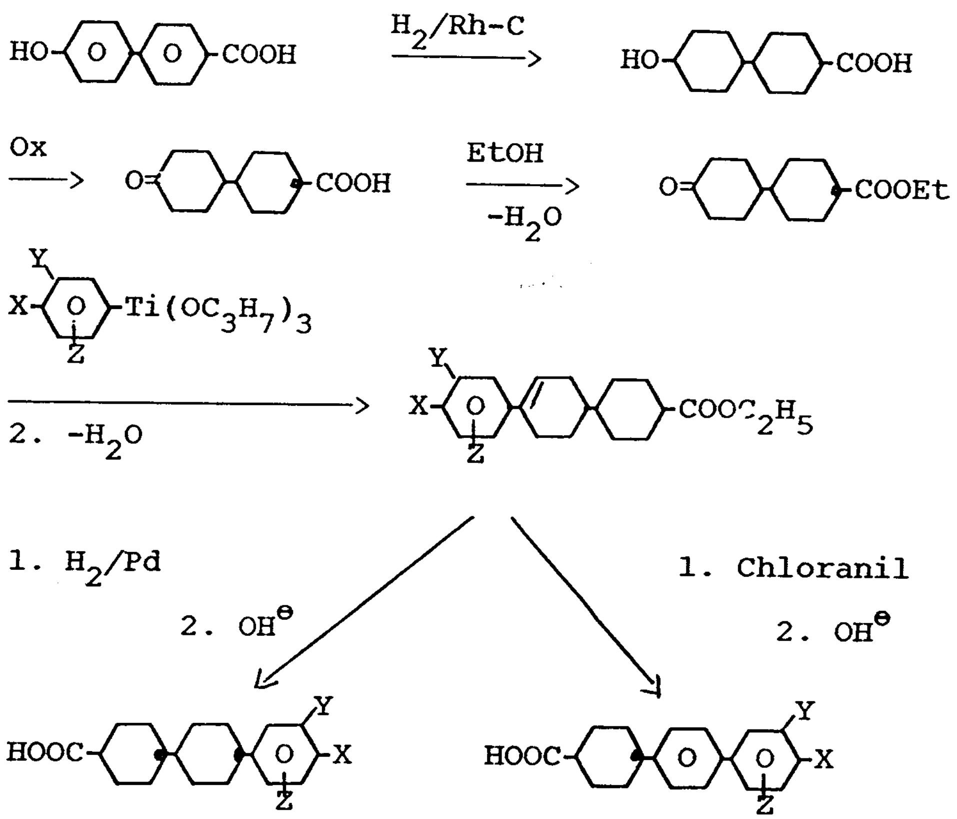

Durch Homologisierung der Cyclohexancarbonsäuren bzw. entsprechender Aldehyde erhält man die erfindungsgemäßen Verbindungen in völliger Analogie zu den weiter oben angegebenen Syntheseschemata.

Die Verbindungen der Formel I mit t = 1 und L = F erhält man in völliger Analogie zum ersten Syntheseschema (Herstellung von Verbindungen mit s = 1 und t = 0) durch Einsatz von

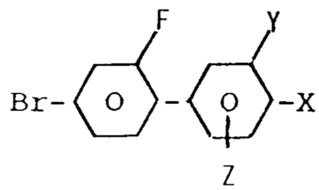

anstelle des Brombenzolderivates. Die Brombiphenyl-Verbindung kann in an sich bekannter Weise durch edelmetallkatalysierte Kopplungsreaktionen hergestellt werden (E. Poetsch, Kontakte (Darmstadt) 1988 (2) S. 15).

Verbindungen mit t = 1 und L = H oder F sind auch durch Pd(0)-katalysierte Kopplungen von Boronsäuren der Formel A

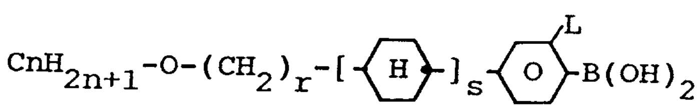

A

mit

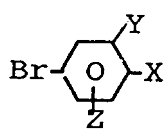

nach Suzuki et al. erhältlich.

Alternativ kann auch

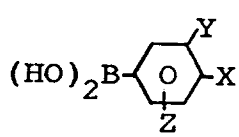

mit entsprechenden Halogeniden, wie z. B. lodiden der Formel B

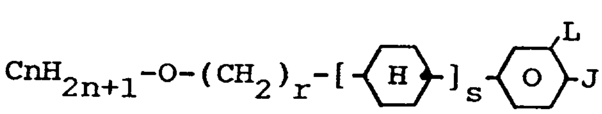

B

gekoppelt werden (L = H oder F).

Die Boronsäuren A sind aus entsprechenden Bromiden erhältlich, die ihrerseits nach Schema 1 hergestellt werden (X = Br, Z = H, Y = F oder H).

Die Jodide B können analog hergestellt werden oder man stellt in völliger Analogie zu Schema 1 aus m-Bromfluorbenzol die Verbindung der Formel

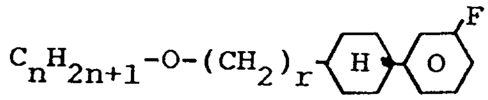

her, die durch ortho-Metallierung mit KOT/BuLi bei -100° und nachfolgende Behandlung mit Jod in B umgewandelt wird.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane und Tolane.

Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-$CH_2CH_2$-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R" voneinander verschieden, wobei einer dieser Reste meist Alkyl oder Alkenyl ist. In den Verbindungen der Teilformeln 1b, 2b, 3b, 4b und 5b bedeutet R" -CN, $-CF_3$, $-OCHF_2$, $-OCF_3$, F, Cl oder -NCS; R hat dabei die bei den Verbindungen der Teilformeln 1a bis 5a angegebene Bedeutung und ist vorzugsweise Alkyl oder Alkenyl. Besonders bevorzugt ist R" ausgewählt aus der Gruppe bestehend aus -F, Cl, $CF_3$, $-OCHF_2$ und $-OCF_3$. Aber auch andere Varianten der vorgesehenen Substituenten in den Verbindungen der Formeln 1, 2, 3, 4 und 5 sind gebräuchlich. Viele

solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:

Gruppe 1: 20 bis 90 %, insbesondere 30 bis 90 %,

Gruppe 2: 10 bis 80 %, insbesondere 10 bis 50 %,

wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

| | |
|---|---|
| DAST | Diethylaminoschwefeltrifluorid |
| DCC | Dicyclohexylcarbodiimid |
| DDQ | Dichlordicyanobenzochinon |
| DIBALH | Diisobutylaluminiumhydrid |
| DMSO | Dimethylsulfoxid |
| KOT | Kalium-tertiär-butanolat |
| THF | Tetrahydrofuran |
| pTSOH | p-Toluolsulfonsäure |

Beispiel 1

Durch Hydrieren von 1-Methoxy-2-[trans-4-[p-trifluormethylphenyl)-cyclohexyl]-ethen [erhältlich aus p-Bromtrifluortoluol nach obigem Syntheseschema] an Pd/C erhält man nach üblicher Aufarbeitung trans-4-Methoxyethyl-(p-trifluormethylphenyl)-cyclohexan.

Beispiel 2 bis 61

Analog Beispiel 1 erhält man aus den entsprechenden Vinylethern die folgenden Verbindungen (Q = $-(CH_2)_r-$,t = 0):

| | r | n | s | X | Y | Z |
|---|---|---|---|---|---|---|
| (2) | 2 | 2 | 1 | $-CF_3$ | H | H |
| (3) | 3 | 1 | 1 | $-CF_3$ | H | H |
| (4) | 4 | 1 | 1 | $-CF_3$ | H | H |

| | r | n | s | X | Y | Z |
|---|---|---|---|---|---|---|
| (5) | 2 | 1 | 1 | F | H | H |
| (6) | 3 | 1 | 1 | F | H | H |
| (7) | 4 | 2 | 1 | F | H | H |
| (8) | 2 | 1 | 1 | Cl | H | H |
| (9) | 3 | 1 | 1 | Cl | H | H |
| (10) | 4 | 2 | 1 | Cl | H | H |
| (11) | 2 | 1 | 1 | $-OCF_3$ | H | H |
| (12) | 3 | 1 | 1 | $-OCF_3$ | H | H |
| (13) | 4 | 2 | 1 | $-OCF_3$ | H | H |
| (14) | 2 | 1 | 1 | $-OCHF_2$ | H | H |
| (15) | 3 | 1 | 1 | $-OCHF_2$ | H | H |
| (16) | 4 | 2 | 1 | $-OCHF_2$ | H | H |
| (17) | 2 | 1 | 1 | -CN | F | H |
| (18) | 3 | 1 | 1 | -CN | F | H |
| (19) | 4 | 2 | 1 | -CN | F | H |
| (20) | 2 | 1 | 1 | -CN | F | F* |
| (21) | 3 | 1 | 1 | -CN | F | F* |
| (22) | 4 | 2 | 1 | -CN | F | F* |
| (23) | 2 | 1 | 1 | F | F | H |
| (24) | 3 | 1 | 1 | F | F | H |
| (25) | 4 | 2 | 1 | F | F | H |
| (26) | 2 | 1 | 1 | Cl | F | H |
| (27) | 3 | 1 | 1 | Cl | F | H |
| (28) | 4 | 2 | 1 | Cl | F | H |
| (29) | 2 | 1 | 1 | $CF_3$ | F | H |
| (30) | 3 | 1 | 1 | $CF_3$ | F | H |
| (31) | 4 | 2 | 1 | $CF_3$ | F | H |
| (32) | 2 | 2 | 2 | $-CF_3$ | H | H |
| (33) | 3 | 1 | 2 | $-CF_3$ | H | H |
| (34) | 4 | 1 | 2 | $-CF_3$ | H | H |
| (35) | 2 | 1 | 2 | F | H | H |
| (36) | 3 | 1 | 2 | F | H | H |
| (37) | 4 | 2 | 2 | F | H | H |

| | r | n | s | X | Y | Z |
|---|---|---|---|---|---|---|
| (38) | 2 | 1 | 2 | Cl | H | H |
| (39) | 3 | 1 | 2 | Cl | H | H |
| (40) | 4 | 2 | 2 | Cl | H | H |
| (41) | 2 | 1 | 2 | $-OCF_3$ | H | H |
| (42) | 3 | 1 | 2 | $-OCF_3$ | H | H, K 51 $S_B$ 105 N 158,8 I |
| (43) | 4 | 2 | 2 | $-OCF_3$ | H | H |
| (44) | 2 | 1 | 2 | $-OCHF_2$ | H | H |
| (45) | 3 | 1 | 2 | $-OCHF_2$ | H | H |
| (46) | 4 | 2 | 2 | $-OCHF_2$ | H | H |
| (47) | 2 | 1 | 2 | -CN | F | H |
| (48) | 3 | 1 | 2 | -CN | F | H |
| (49) | 4 | 2 | 2 | -CN | F | H |
| (50) | 2 | 1 | 2 | -CN | F | F* |
| (51) | 3 | 1 | 2 | -CN | F | F* |
| (52) | 4 | 2 | 2 | -CN | F | F* |
| (53) | 2 | 1 | 2 | F | F | H |
| (54) | 3 | 1 | 2 | F | F | H |
| (55) | 4 | 2 | 2 | F | F | H |
| (56) | 2 | 1 | 2 | Cl | F | H |
| (57) | 3 | 1 | 2 | Cl | F | H |
| (58) | 4 | 2 | 2 | Cl | F | H |
| (59) | 2 | 1 | 2 | $CF_3$ | F | H |
| (60) | 3 | 1 | 2 | $CF_3$ | F | H |
| (61) | 4 | 2 | 2 | $CF_3$ | F | H |

* Z in ortho-Position zu X

Beispiel 62

Man bereitet in THF aus 0,1 mol Magnesium und 0,1 mol p-Bromtrifluormethoxybenzol unter $N_2$-Atmosphäre die entsprechende Grignardverbindung und gibt zu dieser 0,1 mol 4-(trans-4-(4-Oxapentyl)-cyclohexyl)cyclohexanon und rührt das Reaktionsgemisch 1 Stunde in der Siedehitze. Dann wird wie üblich aufgearbeitet und das Reaktionsprodukt am Wasserabscheider durch Kochen mit p-Toluolsulfonsäure in Toluol dehydratisiert. Anschließend wird das Reaktionsprodukt an einem Pd/C-Katalysator hydriert. Die Isomerentrennung erfolgt nach der üblichen basischen Isomerisierung chromatographisch bzw. durch Kristallisation. Man erhält trans,trans-4-(4-Oxapentyl)-4'-(p-trifluormethoxyphenyl)-bicyclohexyl, K 51 $S_B$ 105 N 158.8 I

Beispiel 63

Zu einer Lösung von 0,2 Mol p-(3,4-Difluorphenyl)-phenethyl-alkohol in 150 ml Pyridin wird unter Kühlung eine Lösung von 0,2 Mol p-Toluolsulfonsäurechlorid in 75 ml Toluol so zugetropft, daß die

Reaktionstemperatur 10 °C nicht übersteigt. Nach weiterem Rühren bei Raumtemperatur über Nacht werden 250 ml Toluol zugegeben und nacheinander mit Wasser, 6 N HCl-Lösung, Wasser, 2 N NaOH-Lösung und Wasser gewaschen. Der p-Toluolsulfonsäureester wird nach Entfernen des org. Lösungsmittels durch Umkristallisation gereinigt. Zu einer Natriummethylat-Lösung erhalten aus 250 ml Methanol und 0,6 Mol metallisches Natrium gibt man eine Lösung von 0,4 Mol des erhaltenen p-Toluolsulfonsäureesters in 500 ml Toluol. Nach 3 Stunden Kochen am Rückfluß wird wie üblich aufgearbeitet. Man erhält 4-Methoxyethyl-3',4'-difluorbiphenyl.

Es folgen Beispiele für Medien mit einem Gehalt an mindestens einer Verbindung der Formel I:

Beispiel A

Ein Gemisch aus
7 % trans-4-Methoxyethyl-(p-trifluormethylphenylphenyl)-cyclohexan,
5 % p-(trans-4-Butylcyclohexyl)-benzonitril,
24 % p-(trans-4-Pentylcyclohexyl)-fluorbenzol,
14 % p-(trans-4-Heptylcyclohexyl)-fluorbenzol,
15 % 2-[trans-4-(3,4-Difluorphenyl)-cyclohexyl]-5-butyl-1,3-dioxan,
18 % 2-[trans-4-(p-fluorphenyl)-cyclohexyl]-5-ethyl-1,3-dioxan und
17 % 2-[trans-4-(p-fluorphenyl)-cyclohexyl]-5-propyl-1,3-dioxan
zeigt einen hohen elektrischen Widerstand.

Beispiel B

Ein Gemisch aus
7 % trans-4-Methoxymethyl-(p-trifluormethylphenyl-phenyl)-cyclohexan,
5 % p-(trans-4-Butylcyclohexyl)-benzonitril,
24 % p-(trans-4-Pentylcyclohexyl)-fluorbenzol,
14 % p-(trans-4-Heptylcyclohexyl)-fluorbenzol,
15 % 2-[trans-4-(3,4-Difluorphenyl)-cyclohexyl]-5-butyl-1,3-dioxan,
18 % 2-[trans-4-(p-fluorphenyl)-cyclohexyl]-5-ethyl-1,3-dioxan und
17 % 2-[trans-4-(p-fluorphenyl)-cyclohexyl]-5-propyl-1,3-dioxan
zeigt einen hohen elektrischen Widerstand.

Beispiel C

Ein Gemisch aus
7 % 4-Methoxymethyl-3',4'-difluorbiphenyl,
5 % p-(trans-4-Butylcyclohexyl)-benzonitril,
24 % p-(trans-4-Pentylcyclohexyl)-fluorbenzol,
14 % p-(trans-4-Heptylcyclohexyl)-fluorbenzol,
15 % 2-[trans-4-(3,4-Difluorphenyl)-cyclohexy]-5-butyl-1,3-dioxan,
18 % 2-[trans-4-(p-Fluorphenyl)-cyclohexyl]-5-ethyl-1,3-dioxan und
17 % 2-[trans-4-(p-Fluoryhenyl)-cyclohexyl]-5-propyl-1,3-dioxan
zeigt einen hohen elektrischen Widerstand.

Weitere bevorzugte erfindungsgemäße Verbindungen sind im folgenden angegeben ($Q = (CH_2)_r, L = Z = H, Y = F, X = Cl$):

| n | r | s | t |
|---|---|---|---|
| 1 | 2 | 1 | 0 |
| 1 | 3 | 1 | 0 |
| 1 | 4 | 1 | 0 |
| 1 | 5 | 1 | 0 |

**Patentansprüche**
**Patentansprüche für folgende Vertragsstaaten : DE, GB**

**1.** Benzolderivate der Formel I

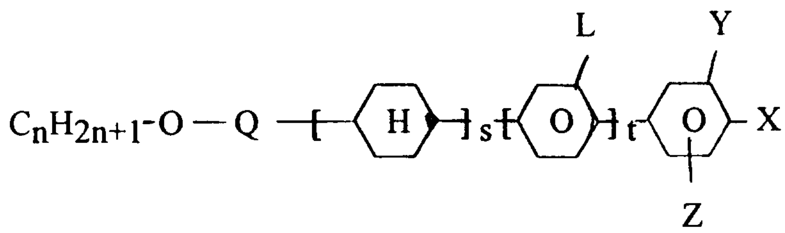

I

worin n 1 bis 7, Q-$(CH_2)_r$-, wobei r 2, 3, 4 oder 5 ist, s 0, 1 oder 2, t 0 oder 1, wobei s + t ≧ 1, X F, Cl, -$CF_3$, -CN, -$OCF_3$ oder -$OCHF_2$ und Y, L und Z jeweils unabhängig voneinander H oder F bedeuten, wobei im Falle X = -CN s 1 oder 2 und/oder Y = F bedeutet, mit der Maßgabe, daß die folgenden Verbindungen ausgeschlossen sind:

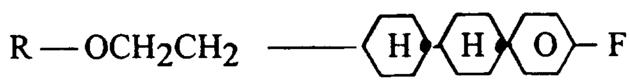

R = geradkettiges Alkyl mit 1 bis 7 C-Atomen

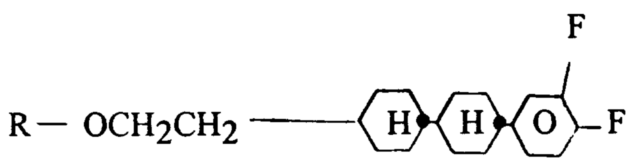

R = geradkettiges Alkyl mit 1 bis 7 C-Atomen

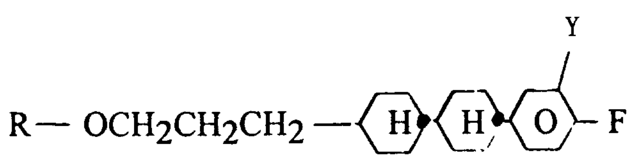

R = geradkettiges Alkyl mit 1 bis 7 C-Atomen
Y = Wasserstoff oder Fluor

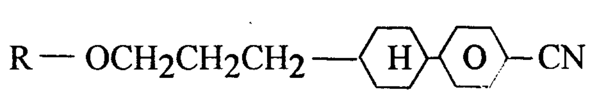

R = geradkettiges Alkyl mit 1 bis 5 C-Atomen

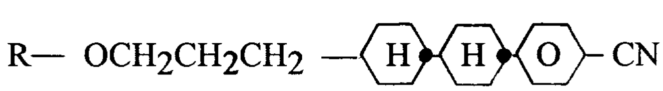

R = geradkettiges Alkyl mit 1 bis 5 C-Atomen

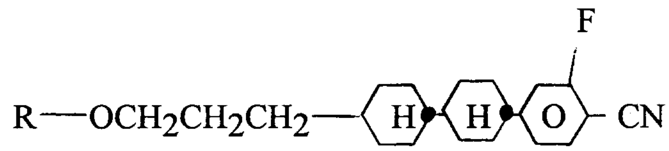

R = geradkettiges Alkyl mit 1 bis 5 C-Atomen

**2.** Benzolderivate nach Anspruch 1, dadurch gekennzeichnet, daß n = 1.

**3.** Benzolderivate nach Anspruch 1 oder 2, dadurch gekennzeichnet, daß X = CN, Y = F und Z = H.

**4.** Benzolderivate nach Anspruch 1 oder 2, dadurch gekennzeichnet, daß X = F, Cl, $-CF_3$, $-OCHF_2$ oder $-OCF_3$ ist.

**5.** Benzolderivate nach Anspruch 4, dadurch gekennzeichnet, daß Y = Z = H.

**6.** Benzolderivate nach Anspruch 4, dadurch gekennzeichnet, daß Y = F und Z = H.

**7.** Verwendung der Benzolderivate der Formel I nach Anspruch 1 als Komponenten flüssigkristalliner Medien für elektrooptische Anzeigen.

**8.** Flüssigkristallines Medium für elektrooptische Anzeigen mit mindestens zwei flüssigkristallinen Komponenten, dadurch gekennzeichnet, daß mindestens eine Komponente ein Benzolderivat der Formel I ist,

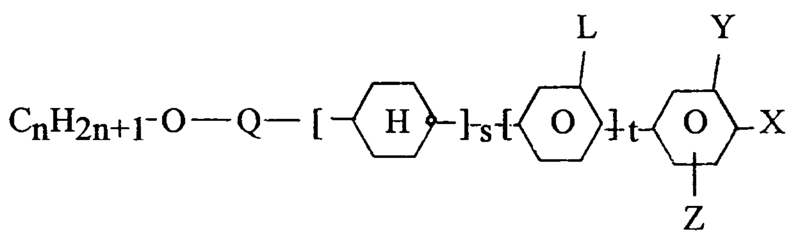

I

worin n 1 bis 7, Q-$(CH_2)_r$-, wobei r = 1, 2, 3, 4 oder 5 ist, s 0, 1 oder 2, t 0 oder 1, wobei s + t ≧ 1, X F, Cl, $-CF_3$, -CN, $-OCF_3$ oder $-OCHF_2$ und Y, L und Z jeweils unabhängig voneinander H oder F bedeuten, und das Medium als weiteren Bestandteil eine oder mehrere Verbindungen der Formeln 1, 2, 3, 4 oder 5 enthält:

R'-L-E-R'' 1

R'-L-COO-E-R'' 2

R'-L-OOC-E-R'' 3

R'-L-$CH_2CH_2$-E-R'' 4

R'-L-C ≡ C-E-R'' 5

worin L und E die gleich oder verschieden sein können, jeweils unabhängig voneinander einen bivalenten Rest aus der aus -Phe-, -Cyc-, -Phe-Phe, -Phe-Cyc-, -Cyc-Cyc-, -Pyr-, -Dio-, -G-Phe-, und -G-Cyc- sowie deren Spiegelbilder gebildeten Gruppen, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten, R' Alkyl, Alkenyl, Alkoxy, Alkenyloxy oder

Alkanoyloxy mit jeweils bis zu 8 Kohlenstoffatomen und R'' F, Cl, $-CF_3$, $-OCHF_2$ oder $-OCF_3$ bedeutet.

**9.** Medium nach Anspruch 8, dadurch gekennzeichnet, daß es ein Benzolderivat der Formel I nach Anspruch 1 enthält.

**10.** Flüssigkristallines Medium für elektrooptische Anzeigen mit mindestens zwei flüssigkristallinen Komponenten, dadurch gekennzeichnet, daß mindestens eine Komponente ein Benzolderivat der Formel I nach Anspruch 1 ist.

**11.** Elektrooptische Anzeige auf der Basis einer Flüssigkristallzelle, dadurch gekennzeichnet, daß die Flüssigkristallzelle ein Medium nach Anspruch 8, 9 oder 10 enthält.

**Patentansprüche für folgende Vertragsstaaten : FR, IT, NL**

**1.** Benzolderivate der Formel I

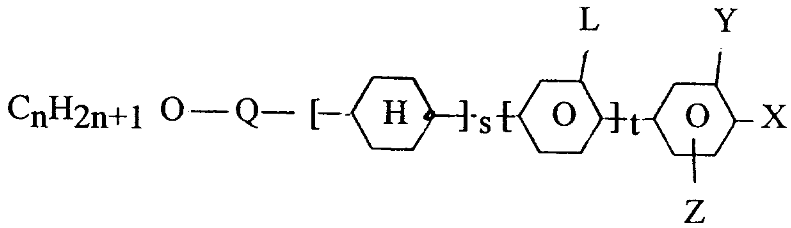

I

worin n 1 bis 7, Q-$(CH_2)$r-, wobei r 2, 3, 4 oder 5 ist, s 0, 1 oder 2, t 0 oder 1, wobei s + t ≧ 1, X F, Cl, $-CF_3$, -CN, $-OCF_3$ oder $-OCHF_2$ und Y, L und Z jeweils unabhängig voneinanderH oder F bedeuten, wobei im Falle X = -CN s 1 oder 2 und/oder Y = F bedeutet.

**2.** Benzolderivate nach Anspruch 1, dadurch gekennzeichnet, daß n = 1.

**3.** Benzolderivate nach Anspruch 1 oder 2, dadurch gekennzeichnet, daß X = CN, Y = F und Z = H.

**4.** Benzolderivate nach Anspruch 1 oder 2, dadurch gekennzeichnet, daß X = F, Cl, $-CF_3$, $-OCHF_2$ oder $-OCF_3$ ist.

**5.** Benzolderivate nach Anspruch 4, dadurch gekennzeichnet, daß Y = Z = H.

**6.** Benzolderivate nach Anspruch 4, dadurch gekennzeichnet, daß Y = F und Z = H.

**7.** Verwendung der Benzolderivate der Formel I nach Anspruch 1 als Komponenten flüssigkristalliner Medien für elektrooptische Anzeigen.

**8.** Flüssigkristallines Medium für elektrooptische Anzeigen mit mindestens zwei flüssigkristallinen Komponenten, dadurch gekennzeichnet, daß mindestens eine Komponente ein Benzolderivat der Formel I ist,

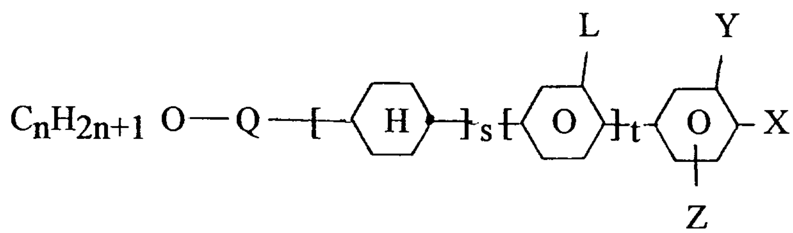

I

worin n 1 bis 7, Q-$(CH_2)$r-, wobei r = 1, 2, 3, 4 oder 5 ist, s 0, 1 oder 2, t 0 oder 1, wobei s + t ≧ 1, X F, Cl, $-CF_3$, -CN, $-OCF_3$ oder $-OCHF_2$ und Y, L und Z jeweils unabhängig voneinander H oder F bedeuten, und das Medium als weiteren Bestandteil eine oder mehrere Verbindungen der Formeln 1, 2,

3, 4 oder 5 enthält:

R'-L-E-R'' 1

R'-L-COO-E-R'' 2

R'-L-OOC-E-R'' 3

R'-L-$CH_2CH_2$-E-R'' 4

R'-L-C ≡ C-E-R'' 5

worin L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe-, und -G-Cyc- sowie deren Spiegelbilder gebildeten Gruppen, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten, R' Alkyl, Alkenyl, Alkoxy, Alkenyloxy oder Alkanoyloxy mit jeweils bis zu 8 Kohlenstoffatomen und R'' F, Cl, $-CF_3$, $-OCHF_2$ oder $-OCF_3$ bedeutet.

**9.** Medium nach Anspruch 8, dadurch gekennzeichnet, daß es ein Benzolderivat der Formel I nach Anspruch 1 enthält.

**10.** Flüssigkristallines Medium für elektrooptische Anzeigen mit mindestens zwei flüssigkristallinen Komponenten, dadurch gekennzeichnet, daß mindestens eine Komponente ein Benzolderivat der Formel I nach Anspruch 1 ist.

**11.** Elektrooptische Anzeige auf der Basis einer Flüssigkristallzelle, dadurch gekennzeichnet, daß die Flüssigkristallzelle ein Medium nach Anspruch 8, 9 oder 10 enthält.

## Claims

## Claims for the following Contracting States : DE, GB

**1.** Benzene derivatives of the formula I

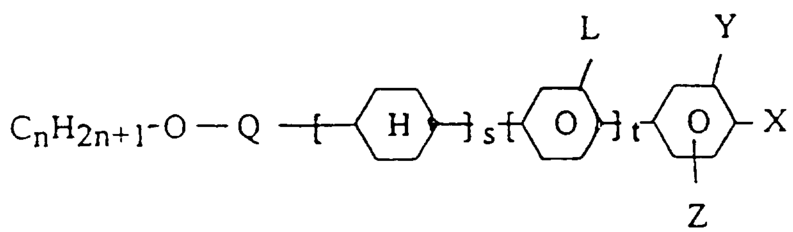

I

in which n is from 1 to 7, Q is $-(CH_2)r-$, where r is 2, 3, 4 or 5, s is 0, 1 or 2, t is 0 or 1, where $s + t \geqq 1$, X is F, Cl, $-CF_3$, -CN, $-OCF_3$ or $-OCHF_2$, and Y, L and Z are in each case, independently of one another, H or F, where, in the case for X = -CN, s is 1 or 2 and/or Y = F, with the proviso that the following compounds are excluded:

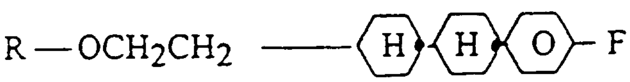

R = straight-chain alkyl having 1 to 7 carbon atoms

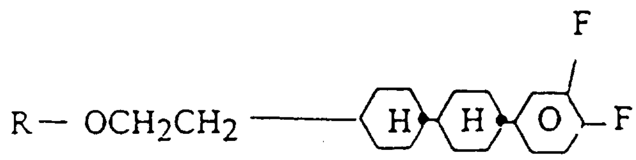

R = straight-chain alkyl having 1 to 7 carbon atoms

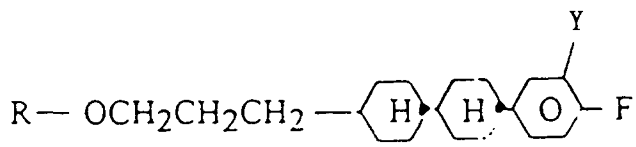

R = straight-chain alkyl having 1 to 7 carbon atoms
Y = hydrogen or fluorine

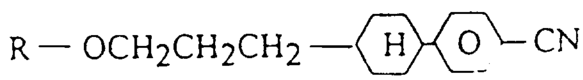

R = straight-chain alkyl having 1 to 5 carbon atoms

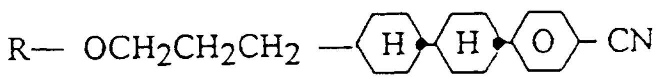

R = straight-chain alkyl having 1 to 5 carbon atoms

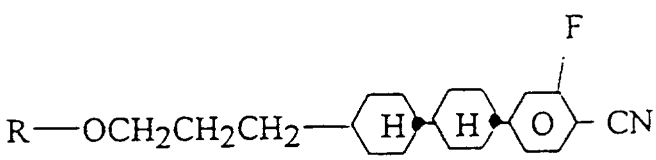

R = straight-chain alkyl having 1 to 5 carbon atoms

**2.** Benzene derivatives according to Claim 1, characterized in that n = 1.

**3.** Benzene derivatives according to Claim 1 or 2, characterized in that X = CN, Y = F and Z = H.

**4.** Benzene derivatives according to Claim 1 or 2, characterized in that X = F, Cl, $-CF_3$, $-OCHF_2$ or $-OCF_3$.

**5.** Benzene derivatives according to Claim 4, characterized in that Y = Z = H.

**6.** Benzene derivatives according to Claim 4, characterized in that Y = F and Z = H.

**7.** Use of the benzene derivatives of the formula I according to Claim 1 as components of liquid-crystalline media for electro-optical displays.

**8.** Liquid-crystalline medium for electro-optical displays, having at least two liquid-crystalline components, characterized in that at least one component is a benzene derivative of the formula I,

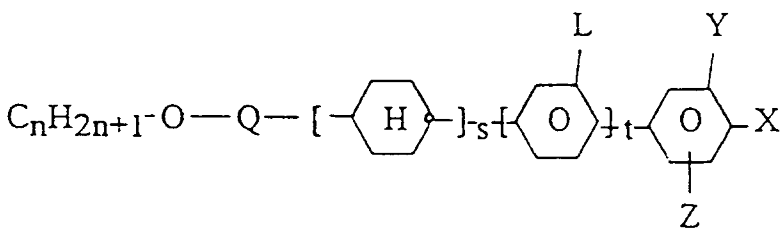

I

in which n is from 1 to 7, Q is $-(CH_2)r-$, where r is 1, 2, 3, 4 or 5, s is 0, 1 or 2, t is 0 or 1, where $s+t \geqq 1$, X is F, Cl, $-CF_3$, -CN, $-OCF_3$ or $-OCHF_2$, and Y, L and Z are each, independently of one another, H or F, and the medium contains, as further constituents, one or more compounds of the formulae 1, 2, 3, 4 or 5:

R'-L-E-R" 1

R'-L-COO-E-R" 2

R'-L-OOC-E-R" 3

R'-L-$CH_2CH_2$-E-R" 4

R'-L-C ≡ C-E-R" 5

in which L and E, which may be identical or different, are in each case, independently of one another, a divalent radical from the group formed by -Phe-, -Cyc-, -Phe-Phe-, -Phe-Cyc-, -Cyc-Cyc-, -Pyr-, -Dio-, -G-Phe-and -G-Cyc-, and their mirror images, where Phe is unsubstituted or fluorine-substituted 1,4-phenylene, Cyc is trans-1,4-cyclohexylene or 1,4-cyclohexenylene, Pyr is pyrimidine-2,5-diyl or pyridine-2,5-diyl, Dio is 1,3-dioxane-2,5-diyl and G is 2-(trans-1,4-cyclohexyl)ethyl, pyrimidine-2,5-diyl, pyridine-2,5-diyl or 1,3-dioxane-2,5-diyl, R' is alkyl, alkenyl, alkoxy, alkenyloxy or alkanoyloxy, each having up to 8 carbon atoms and R" is F, Cl, $-CF_3$, $-OCHF_2$ or $-OCF_3$.

**9.** Medium according to Claim 8, characterized in that it contains a benzene derivative of the formula I according to Claim 1.

**10.** Liquid-crystalline medium for electro-optical displays, having at least two liquid-crystalline components, characterized in that at least one component is a benzene derivative of the formula I according to Claim 1.

**11.** Electro-optical display based on a liquid-crystal cell, characterized in that the liquid-crystal cell contains a medium according to Claim 8, 9 or 10.

**Claims for the following Contracting States : FR, IT, NL**

**1.** Benzene derivatives of the formula I

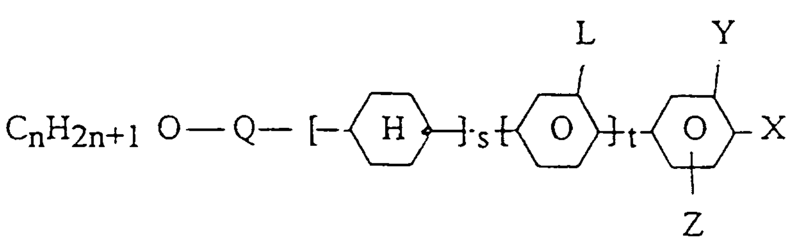

I

in which n is from 1 to 7, Q is $-(CH_2)r-$, where r is 2, 3, 4 or 5, s is 0, 1 or 2, t is 0 or 1, where $s+t \geqq 1$,

X is F, Cl, $-CF_3$, -CN, $-OCF_3$ or $-OCHF_2$, and Y, L and Z are in each case, independently of one another, H or F, where, in the case for X = -CN, s is 1 or 2 and/or Y = F.

**2.** Benzene derivatives according to Claim 1, characterized in that n = 1.

**3.** Benzene derivatives according to Claim 1 or 2, characterized in that X = CN, Y = F and Z = H.

**4.** Benzene derivatives according to Claim 1 or 2, characterized in that X = F, Cl, $-CF_3$, $-OCHF_2$ or $-OCF_3$.

**5.** Benzene derivatives according to Claim 4, characterized in that Y = Z = H.

**6.** Benzene derivatives according to Claim 4, characterized in that Y = F and Z = H.

**7.** Use of the benzene derivatives of the formula I according to Claim 1 as components of liquid-crystalline media for electro-optical displays.

**8.** Liquid-crystalline medium for electro-optical displays, having at least two liquid-crystalline components, characterized in that at least one component is a benzene derivative of the formula I,

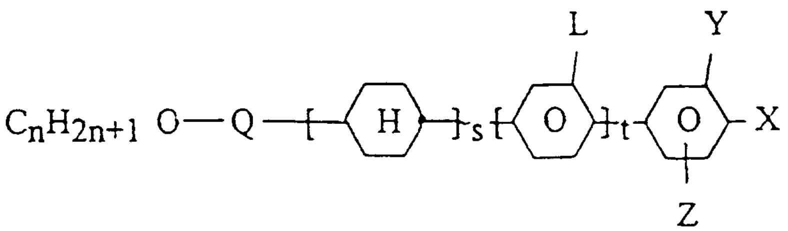
I

in which n is from 1 to 7, Q is $-(CH_2)r-$, where r is 1, 2, 3, 4 or 5, s is 0, 1 or 2, t is 0 or 1, where $s+t \geq 1$, X is F, Cl, $-CF_3$, -CN, $-OCF_3$ or $-OCHF_2$, and Y, L and Z are each, independently of one another, H or F, and the medium contains, as further constituents, one or more compounds of the formulae 1, 2, 3, 4 or 5:

R'-L-E-R'' 1

R'-L-COO-E-R'' 2

R'-L-OOC-E-R'' 3

R'-L-$CH_2CH_2$-E-R'' 4

R'-L-C ≡ C-E-R'' 5

in which L and E, which may be identical or different, are in each case, independently of one another, a divalent radical from the group formed by -Phe-, -Cyc-, -Phe-Phe-, -Phe-Cyc-, -Cyc-Cyc-, -Pyr-, -Dio-, -G-Phe-and -G-Cyc-, and their mirror images, where Phe is unsubstituted or fluorine-substituted 1,4-phenylene, Cyc is trans-1,4-cyclohexylene or 1,4-cyclohexenylene, Pyr is pyrimidine-2,5-diyl or pyridine-2,5-diyl, Dio is 1,3-dioxane-2,5-diyl and G is 2-(trans-1,4-cyclohexyl)ethyl, pyrimidine-2,5-diyl, pyridine-2,5-diyl or 1,3-dioxane-2,5-diyl, R' is alkyl, alkenyl, alkoxy, alkenyloxy or alkanoyloxy, each having up to 8 carbon atoms and R'' is F, Cl, $-CF_3$, $-OCHF_2$ or $-OCF_3$.

**9.** Medium according to Claim 8, characterized in that it contains a benzene derivative of the formula I according to Claim 1.

**10.** Liquid-crystalline medium for electro-optical displays, having at least two liquid-crystalline components, characterized in that at least one component is a benzene derivative of the formula I according to Claim

1.

**11.** Electro-optical display based on a liquid-crystal cell, characterized in that the liquid-crystal cell contains a medium according to Claim 8, 9 or 10.

**Revendications**

**Revendications pour les Etats contractants suivants : DE, GB**

**1.** Dérivés benzéniques de formule I

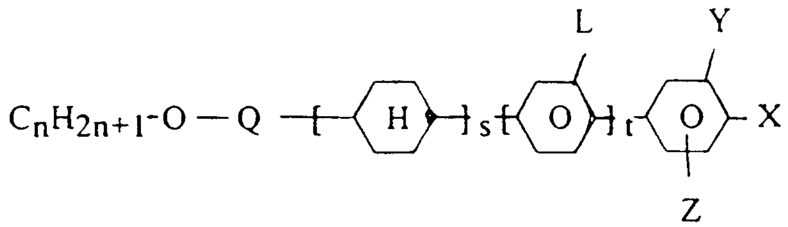

I

dans laquelle n est un nombre allant de 1 à 7, Q représente $-(CH_2)_r-$, r étant égal à 2, 3, 4 ou 5, s est égal à 0, 1 ou 2, t à 0 ou 1, la somme s + t étant supérieure ou égale à 1, X représente F, Cl, $CF_3$, -CN, - $OCF_3$ ou $-OCHF_2$ et Y, L et Z représentent chacun, indépendamment les uns des autres, H ou F, sous réserve que lorsque X représente -CN, s est égal à 1 ou 2 et/ou Y représente F, les composés suivants étant exclus :

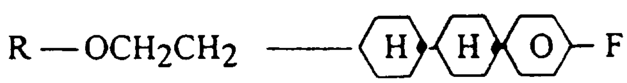

R = groupe alkyle à chaîne droite en C 1-C 7

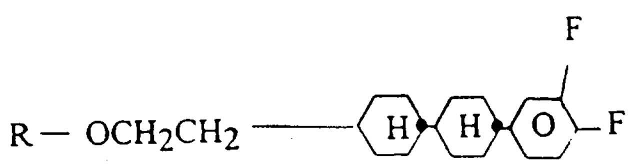

R = groupe alkyle à chaîne droite en C 1-C 7

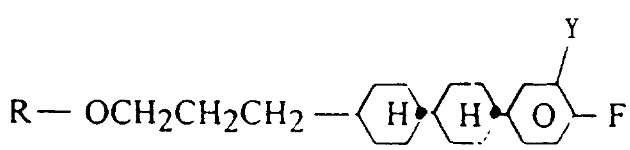

R = groupe alkyle à chaîne droite en C 1-C 7,

et

Y = hydrogène ou fluor

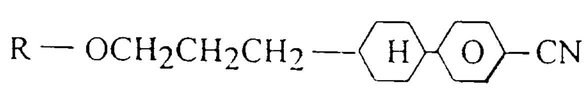

R = groupe alkyle à chaîne droite en C 1-C 5

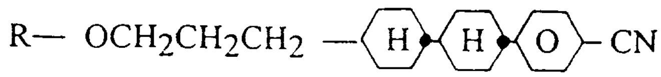

R = groupe alkyle à chaîne droite en C 1-C 5

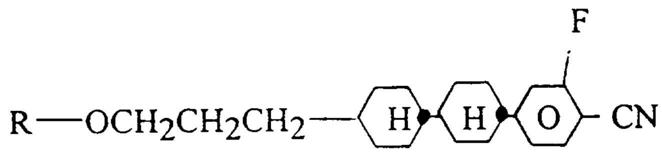

R = groupe alkyle à chaîne droite en C 1-C 5

**2.** Dérivés benzéniques selon revendication 1, caractérisés en ce que n = 1.

**3.** Dérivés benzéniques selon revendication 1 ou 2, caractérisés en ce que X = CN, Y = F et Z = H.

**4.** Dérivés benzéniques selon revendication 1 ou 2, caractérisés en ce que X = F, Cl, $-CF_3$, $-OCHF_2$ ou $-OCF_3$.

**5.** Dérivés benzéniques selon revendication 4, caractérisés en ce que Y = Z = H.

**6.** Dérivés benzéniques selon revendication 4, caractérisés en ce que Y = F et Z = H.

**7.** Utilisation des dérivés benzéniques de formule I selon revendication 1, en tant que composants de milieux à cristaux liquides pour affichages électro-optiques.

**8.** Milieu à cristaux liquides pour affichages électro-optiques, contenant au moins deux composants à cristaux liquides et caractérisé en ce qu'un composant au moins est un dérivé benzénique de formule I

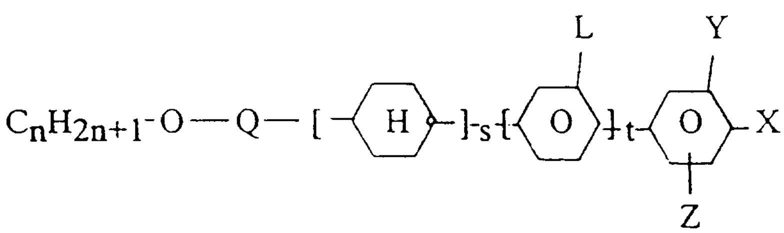

I

dans laquelle n est un nombre allant de 1 à 7, Q représente $-(CH_2)_r-$, r étant égal à 1, 2, 3, 4 ou 5, s est égal à 0, 1 ou 2, t à 0 ou 1, et la somme s + t est supérieure ou égale à 1, X représente F, Cl, $-CF_3$, -CN, $-OCF_3$ ou $-OCHF_2$, et Y, L et Z représentent chacun, indépendamment les uns des autres, H ou F, le milieu contenant en tant qu'autres constituants un ou plusieurs composés de formules 1, 2, 3, 4 ou 5 :

R'-L-E-R'' 1

R'-L-COO-E-R'' 2

R'-L-OOC-E-R'' 3

R'-L-$CH_2CH_2$-E-R'' 4

R'-L-C ≡ C-E-R'' 5

dans lesquelles L et E, ayant des significations identiques ou différentes, représentent chacun, indépendamment l'un de l'autre, un radical bivalent choisi parmi -Phe-, -Cyc-, -Phe-Phe-, Phe-Cyc-, Cyc-Cyc-, -Pyr-, -Dio-, -G-Phe- et -G-Cyc- et leurs images spéculaires, Phe représentant un groupe 1,4-phénylène non substitué ou substitué par le fluor, Cyc un groupe trans-1,4-cyclohexylène ou 1,4-cyclohexénylène, Pyr un groupe pyrimidine-2,5-diyle ou pyridine-2,5-diyle, Dio le groupe 1,3-dioxanne-2,5-diyle et G un groupe 2-(trans-1,4-cyclohexyl)-éthyle, pyrimidine-2,5-diyle, pyridine-2,5-diyle ou 1,3-dioxanne-2,5-diyle, R' représente un groupe alkyle, alcényle, alcoxy, alcényloxy ou alcanoyloxy contenant chacun jusqu'à 8 atomes de carbone et R'' représente F, Cl, $-CF_3$, $-OCHF_2$ ou $-OCF_3$.

**9.** Milieu selon revendication 8, caractérisé en ce qu'il contient un dérivé benzénique de formule I selon revendication 1.

**10.** Milieu à cristaux liquides pour affichages électro-optiques contenant au moins deux composants à cristaux liquides, caractérisé en ce qu'au moins un composant est un dérivé benzénique de formule I selon revendication 1.

**11.** Affichages électro-optiques à base d'une cellule à cristaux liquides, caractérisés en ce que la cellule à cristaux liquides contient un milieu selon revendication 8, 9 ou 10.

**Revendications pour les Etats contractants suivants : FR, IT, NL**

**1.** Dérivés benzéniques de formule I

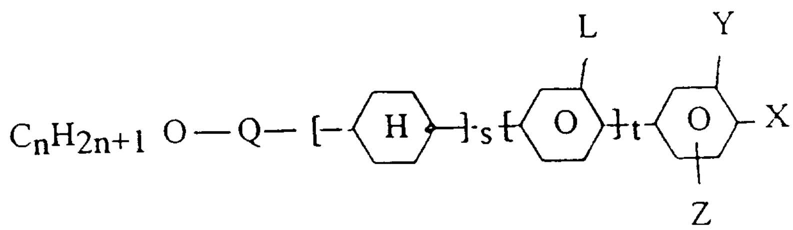

I

dans laquelle n est un nombre allant de 1 à 7, Q représente $-(CH_2)_r-$, r étant égal à 2, 3, 4 ou 5, s est égal à 0, 1 ou 2, t à 0 ou 1 et la somme s + t est supérieure ou égale à 1, X représente F, Cl, $-CF_3$, -CN, $-OCF_3$ ou $-OCHF_2$, et Y, L et Z représentent chacun, indépendamment les uns des autres, H ou F, sous réserve que lorsque X représente -CN, s est égal à 1 ou 2 et/ou Y = F.

**2.** Dérivés benzéniques selon revendication 1, caractérisés en ce que n = 1.

**3.** Dérivés benzéniques selon revendication 1 ou 2, caractérisés en ce que X = CN, Y = F et Z = H.

**4.** Dérivés benzéniques selon revendication 1 ou 2, caractérisés en ce que X = F, Cl, $-CF_3$, $-OCHF_2$ ou -$OCF_3$.

**5.** Dérivés benzéniques selon revendication 4, caractérisés en ce que Y = Z = H.

**6.** Dérivés benzéniques selon revendication 4, caractérisés en ce que Y = F et Z = H.

**7.** Utilisation des dérivés benzéniques de formule I selon revendication 1 en tant que composants de milieux à cristaux liquides pour affichages électro-optiques.

**8.** Milieu à cristaux liquides pour affichages électro-optiques, contenant au moins deux composants à cristaux liquides, caractérisé en ce qu'au moins un composant est un dérivé benzénique de formule I

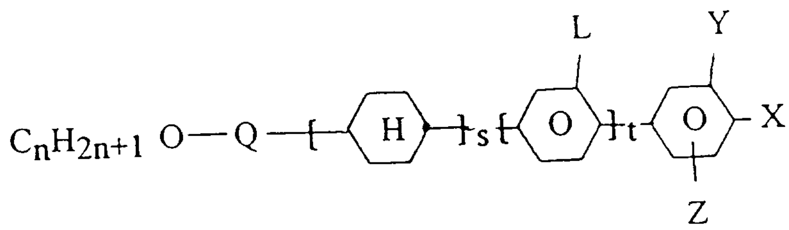

I

dans laquelle n est un nombre allant de 1 à 7, Q représente $-(CH_2)_r-$, r étant égal à 1, 2, 3, 4 ou 5, s est égal à 0, 1 ou 2, t à 0 ou 1 et la somme s + t est supérieure ou égale à 1, X représente F, Cl, $-CF_3$, -CN, $-OCF_3$ ou $-OCHF_2$, et Y, L et Z représentent chacun, indépendamment les uns des autres, H ou F, et en ce que le milieu contient en tant qu'autres constituants un ou plusieurs composés de formules 1, 2, 3, 4 ou 5 :

R'-L-E-R" 1

R'-L-COO-E-R" 2

R'-L-OOC-E-R" 3

R'-L-$CH_2CH_2$-E-R" 4

R'-L-C ≡ C-E-R" 5

dans lesquelles L et E, ayant des significations identiques ou différentes, représentent chacun, indépendamment l'un de l'autre, un radical bivalent choisi parmi -Phe-, -Cyc-, -Phe-Phe-, -Phe-Cyc-, -Cyc-Cyc-, -Pyr-, -Dio-, -G-Phe- et -G-Cyc-, et leurs images spéculaires, Phe représentant un groupe 1,4-phénylène non substitué ou substitué par le fluor, Cyc un groupe trans-1,4-cyclohexylène ou 1,4-cyclohexénylène, Pyr un groupe pyrimidine-2,5-diyle ou pyridine-2,5-diyle, Dio un groupe 1,3-dioxanne-2,5-diyle et G un groupe 2-(trans-1,4-cyclohexyl)-éthyle, pyrimidine-2,5-diyle, pyridine-2,5-diyle ou 1,3-dioxanne-2,5-diyle, R' un groupe alkyle, alcényle, alcoxy, alcényloxy ou alcanoyloxy contenant chacun jusqu'à 8 atomes de carbone et R" représente F, Cl, $-CF_3$, $-OCHF_2$ ou $-OCF_3$.

**9.** Milieu selon revendication 8, caractérisé en ce qu'il contient un dérivé benzénique de formule I selon revendication 1.

**10.** Milieu à cristaux liquides pour affichages électro-optiques à au moins deux composants à cristaux liquides, caractérisé en ce qu'au moins un composant est un dérivé benzénique de formule I de la revendication 1.

**11.** Affichages électro-optiques à base d'une cellule à cristaux liquides, caractérisés en ce que la cellule à cristaux liquides contient un milieu selon revendication 8, 9 ou 10.